# EUROPEAN PATENT SPECIFICATION

(11) **EP 1 575 465 B1**
(45) Date of publication and mention of the grant of the patent: **15.12.2010**
(21) Application number: 03800176.4
(22) Date of filing: 24.12.2003
(51) Int. Cl.: A61F 6/22, A61F 2/01

(54) **CONTRACEPTIVE DEVICE**
EMFÄNGNISVERHÜTUNGSVORRICHTUNG
DISPOSITIF CONTRACEPTIF

(30) Priority: 24.12.2002 US 436722 P
(43) Date of publication of application: 21.09.2005
(73) Proprietor: Conceptus, Inc., Mountain View, CA 94041 (US)
(72) Inventor: TREMULIS, William, S., Redwood City, CA 94065 (US); CALLISTER, Jeffrey, P., Redwood City, CA 94061 (US)
(74) Representative: Bates, Alan Douglas Henry
(86) International application number: PCT/US2003/041275
(87) International publication number: WO 2004/058109

(56) References cited:
- EP-A- 1 433 437
- DE-A- 3 038 928
- US-A- 3 334 629
- US-A- 4 579 110
- US-A- 4 688 553
- US-A- 5 370 657
- US-A- 5 669 933

## Description

### BACKGROUND OF THE INVENTION

This invention generally relates to the field of occluding devices, delivery systems for such devices and the method of using such devices and systems in the occlusion of body passageways. The invention is particularly useful for the occluding reproductive lumens such as a female patient's fallopian tubes or a male patient's vas deferens to affect contraception. Although the occlusion of a patient's reproductive lumens will be discussed herein in detail, it can be appreciated that the devices, methods and systems described herein can easily be adapted to occlude a patient's arteries or veins in a variety of situations. the nidus of an arterial-venous malformation, patent ductus arteriosis in infants, as well as feeding arteries to cancerous tumors, among other passageways. The invention also provides means for delivering vessel supporting devices such as coronary stents or venous' or arterial embolic filters, to the desired location through a steerable system. Those skilled in the art will immediately recognize that various combinations, modifications, and equivalents of the inventions described herein can be used without departing from the scope of these inventions.

Conventional contraceptive strategies generally fall within three categories: physical barriers, drugs and surgery. While each have certain advantages, they also suffer from various drawbacks. Barriers such as condoms and diaphragms are subject to failure due to breakage, displacement and misplacement. Drug strategies, such as the pill and Norplant™, which rely on artificially controlling hormone levels, suffer from known and unknown side-effects from prolonged use. Surgical procedures, such as tubal ligation and vasectomy, are very effective, but involve the costs and attendant risks of surgery, and are frequently not reversible.
US 4,688,553 and US 5,669,933 disclose blood clot filters that are to be placed in the blood stream for the trapping of clots to prevent such clots travelling to the heart or lungs of the patient.
US 3,334.629 discloses an occlusive device for restricting the flow of blood through a blood vessel particularly for use in the vena cava to control circulation of venus blood to the heart.
DE 3038928 and US 4,579,110 disclose tubular pessaries for blocking the uterine tube.
EP 1433437 discloses a barrier device for covering the ostium of a left atrial appendage. The device includes a membrane that has an outer periphery with a dimension larger than a corresponding dimension of the ostium. The membrane prevents blood clots in the atrial appendage from escaping there from and entering the blood stream.

### SUMMARY OF THE INVENTION

The invention provides an occluding member for a patient's reproductive lumen as defined in the appended independent claim to which reference should now be made. Preferred or advantageous features of the invention are defined in dependent sub-claims.

The present invention is directed to occlusion devices for occluding reproductive body lumens such as a female's fallopian tubes and a male's vas deferens.

The occlusion device embodying features of the invention has at least one segment with a plurality of expansive elements, preferably self-expanding, secured by one end thereof to a central location within the device. The first segment has a first expansive element with a first secured end and a second free end raically spaced from the first end when in an expanded configuration. The first segment preferably has at least one additional expansive element having a first secured end and a second free end radially spaced from the first end in the expanded configuration. Preferably expansive elements are equally spaced about the central location of each segment with the first secured ends of the expansive elements being secured at the central location.

The occlusion member may have one or more self expanding expansive spider-like segments (hereinafter spider segments). A plurality of spider segments are preferably axially aligned and secured together by connecting members. Specifically, the occluding member may have a first spider segment at a first end of the device, a second spider segment at a second end of the device. In further embodiments, the occluding device may have at least one intermediate spider segment between the first and second spider segments. The self expansive spider devices are preferably secured together by connecting members such as straight beams or curvilinear structures such as S-shape or Z-shape members. Connecting members having other shapes may also be employed.

The expansive elements of the spider segments may have a first section extending from the first end of the element which is oriented toward a first end of the occluding member and a second section extending to the second end of the expansive element which is oriented to a second end of the occluding member. The sections of the expansive elements may be straight or curved or have other shapes. The orientation of the expansive elements may alternate so that the first section of one expansive element of a spider segment is oriented in a first direction toward one end of the device and the first section of another expansive element of the same spider segment is oriented in a second direction toward a second end of the device. Additionally, the second section of a first expansive element may be oriented toward the second end of the occluding member and the second section of a second expansive element is oriented toward a second end of the occluding member. Alternatively, the expansive elements of one spider segment may be oriented in one direction and the expansive elements of another spider element may be in a second, (e.g. opposite) direction. The angle between the first and second sections of the expansive elements may be varied to allow for sizing the expanded configuration of the occluding device.

The occluding device may be delivered to an intracorporeal location through a delivery system which has a delivery catheter with an inner lumen configured to receive the occluding device in a constricted configuration, where the expansive elements of the one or more spider segments of the occluding device are radially compressed. A pusher element is slidably disposed within the inner lumen of the delivery catheter and has a distal end or head configured to engage the proximal end of the constricted occluding device and urge the occluding device out a discharge port in the distal end of the catheter. The pusher element is configured so that the proximal end thereof will extend out of the patient when deploying the occluding device to facilitate the manipulation of the pusher element. Because the occluding device is capable of being compressed to a very low profile, the delivery catheter may be restricted to very small transverse dimensions. Suitable delivery catheters may have an inner diameter of about 0.008 to about 0.08 inch (0.2-2.00 mm), preferably about 0.015 to about 0.025 inch (0.4-0.6 mm). The smaller diameter delivery catheters reduce the pain and discomfort of delivering the occluding device to the intracorporeal location within the patient. Moreover, the small diameter catheter greatly increase the locations which these occluding devices can be deployed.

The spider segments of the occluding devices embodying features of the invention, which are suitable for implantation within a female patient's fallopian tubes, have expanded transverse dimensions of about 1 to about 5 mm, preferably about 2 to about 4 mm. The length of the occluding device for such uses may range from about 0.2 to about 3 inch (0.5-7.6 cm), preferably about 0.7 to about 1.5 inch (1.8-3.8 cm). Spacing between spider segments is usually selected to ensure that expansion and contraction of the spider segments do not interfere with the expansion and contraction of adjacent segments. Typically, an intrasegment spacing of about 0.1 to about 1 mm, preferably about 0.2 to about 0.8 mm as measured in the collapsed configuration. Additionally, the spider segment spacing of the device should not interfere with advancement and delivery of the device. Uses in other treatments and other intracorporeal locations may require different size occluding devices. About 1 to about 12, preferably about 3 to about 6 spider segments may be disposed along the length of the occluding device.

The occluding device according to the invention is provided with a material to facilitate tissue growth within the occluding device to effect lumen occlusion. Suitable materials include fibrous synthetic materials such as Dacron or Nylon and other materials such as collagen, tissue matrix or other material which encourages or supports tissue ingrowth. The fibrous materials may be deployed about or between the expansive members of the spider segments or the connectors between the spider segments. The various components of the occluding devices may be provided with porous jackets or surfaces for the same purpose.

The invention has numerous advantages over the art. The configuration of this invention provides for an occluding device that may be compressed into a very small diameter and delivered through a delivery catheter of very low profile. This allows for delivery systems with improved ease of use and the ability to use this device in combination with other devices where that would not be possible with an occlusion device of larger diameter. It provides for an expandable device that, once expanded and placed, may be very stationary and stable. If used in combination with other devices and attached to other deices, the occluding device may provide an excellent stable and stationary reference point or anchor when place in the tissue. The advantageous stationary reference point or anchor when placed in the tissue. The advantageous configuration provides an excellent drug delivery platform. Because of the configuration, the device is inexpensive and easy to manufacture. The use of a combination of subcomponents makes the over-all occlusive device highly versatile and adjustable to a great variety of advantageous configurations with the subcomponents widely spaced, or close together, or numerous, or few in number, depending on the desired use. It is highly efficient in its configuration, and otherwise very adaptable in ways that will be clear to one of skill in the art in view of the drawings and detailed description contained herein.

The delivery catheter may be of an over the wire (OTW) or of rapid exchange (RX) type design. An OTW catheter has a guide wire lumen extending the full length of the catheter, whereas an RX type catheter has a relatively short guide wire lumen in a distal portion of the catheter. With a rapid exchange type catheter, the guide wire lumen (as measured from a distal guide wire port to a proximal guide wire port) is about 0.5 to about 50 cm, typically about 10 to about 35 cm.

The alternative means of using a pushing device proximal to the collapsed device allows for the device to have a very small collapsed profile since no guide wire needs to pass through it, however such systems do not allow for any steerability of the system through the body lumens. For these reasons and others it would be desirable to have a small diameter system that still allows for steerability of the guide wire while advancing through the body passageways.

Although these procedures all may benefit from the inventions described herein, one particularly useful and immediate benefit for these devices, methods and systems is in the delivery of occlusion devices to the fallopian tubes for contraceptive purposes. At least some of these objectives will be met by the novel inventions, devices, methods and systems described hereinbelow.

### BRIEF DESCRIPTION OF THE DRAWINGS

Figure 1 is an elevational view of an occluding device having a single spider segment.

Figure 2 is an end view of the occluding device shown in Figure 1.

Figure 3 is an elevational view of an occluding device having a plurality of interconnected spider segments in expanded configurations.

Figure 4 is an elevational view of the occluding device shown in Figure 3 compressed into a contracted configuration.

Figure 5 is an elevational view, partially is section of a rapid exchange-type delivery catheter illustrating the advancement of an occluding device embodying features of the invention.

Figures 6 and 7 are transverse cross-sectional views of the delivery catheter and guide wire shown in Figure 5 taken along the lines 6-6 and 7-7 respectfully.

Figure 8 is an elevational view of an over-the-wire type delivery catheter.

Figure 9 is a transverse cross-section of the over-the-wire delivery catheter shown in Figure 8, taken along the lines 9-9.

Figure 10 is an elevational view, partially in section, of the distal section of the over-the-wire delivery catheter shown in Figure 8 illustrating the advancement of an occluding device embodying features of the invention within the inner lumen of the delivery catheter by a pusher element after the guidewire has been withdrawn.

Figure 11 is an elevational view, partially in section, of an over-the-wire delivery catheter with a combined guide wire-pusher element advancing an occluding member embodying features of the invention through the inner lumen of the catheter.

Figure 12 is a transverse cross-sectional view of the delivery catheter shown in Figure 11 taken along the lines 12-12

Figure 13 is an elevational view of an occlusion device embodying features of the invention disposed within a body lumen such as a female's fallopian tube.

Figure 14 is a partial elevational view of the occluding device shown in Figure 5 with fibrous material disposed about the expansive elements and a connecting member.

### DETAILED DESCRIPTION OF EMBODIMENTS OF THE INVENTION

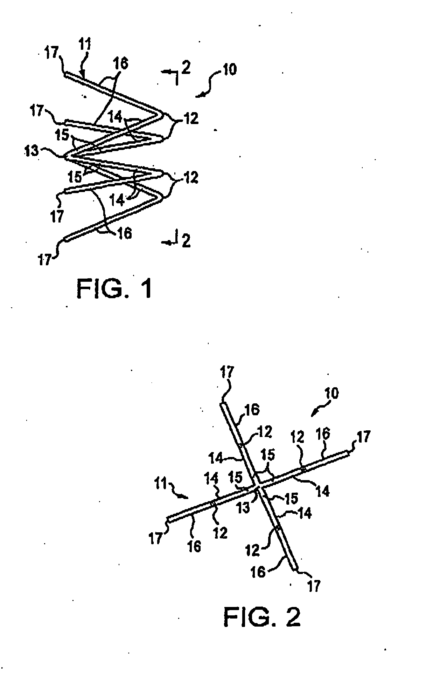

Figures 1 and 2 illustrate an occluding device 10 which is suitable to occlude a patient's reproductive lumen. The occluding 10 is in the form of a spider segment 11 that has a plurality of expansive elements 12 which radiate from a central location 13. The expansive elements have first sections 14 with a first end 15 secured to the central location 13 and second sections 16 with free ends 17 radially displaced from the central location 13 in the expanded configuration as shown. The central location 13 need not be the geometric center of the device 10. For example, it may be off set from the geometric center and be provided with expansive elements of different lengths.

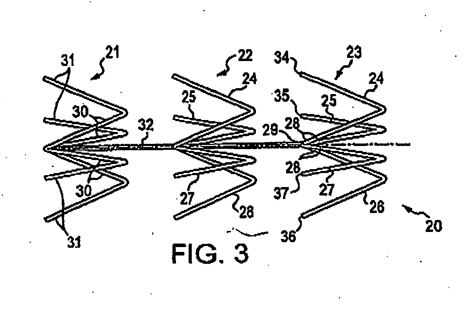

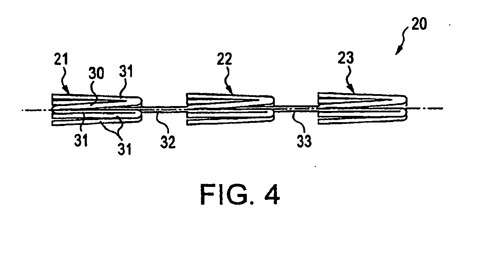

Figure 3 represents an elevational view of an occlusion device 20 with three spider segments 21, 22 and 23 that have the same structure as the spider segment 11 shown in Figures 1 and 2. The individual spider segments 21-23 have expansive elements 24, 25, 26 and 27 which are secured by a first ends 28 to the central location 29. Each expansive element of a spider segment has a first section 30 which is adjacent to the central location or center line axis 29 and which is oriented toward one end of the occluding member 20 and a second section 31 which is oriented toward the other end of the occluding device 20. The angle between the first and second sections 30 and 31 of the expansive elements ranges from about 20° to about 75°, preferably about 30° to about 60°. The spider segments 21 and 22 are interconnected by beam 32 and spider segments 22 and 23 are interconnected by beam 33 both of which lie along the center line axis 29. The free end 33, 34 and 35 of the expansive elements 24-27 are configured to engage the interior body lumen and seat the occlusion device therein. Figure 3 illustrates the occlusion device 20 in an expanded configuration and Figure 4 illustrates the device 20 compressed into a constricted configuration with the first and second sections 30 and 31 of the expansive elements 24-27 folded together so as to present a smaller profile.

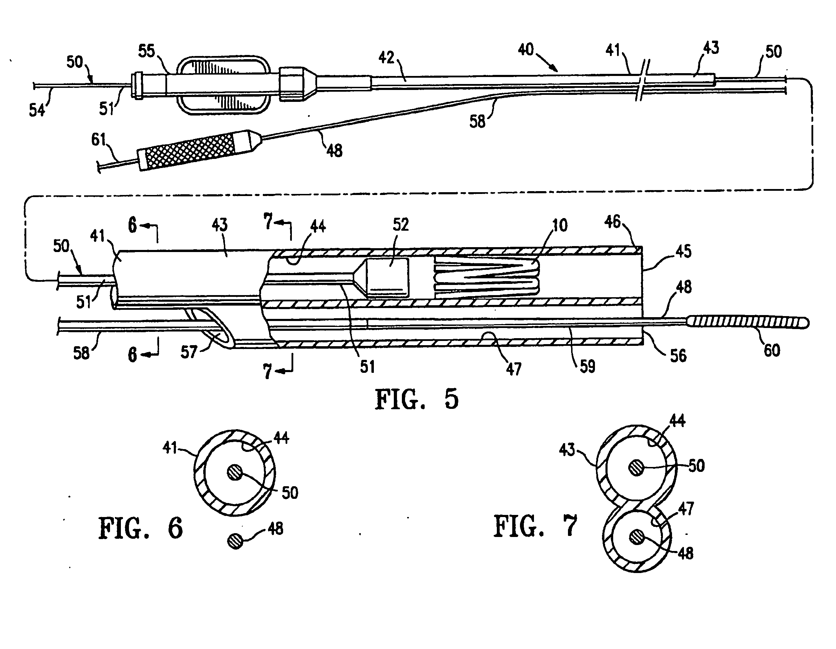

Figures 5-7 shows a rapid exchange delivery catheter 40 suitable to deliver an occluding member 10 as shown in Figure 1. The delivery catheter 40 has an elongated shaft 41 with a proximal shaft section 42 and a distal shaft section 43. The elongated shaft 41 has a lumen 44 which extends the length of the shaft to the discharge port 45 in the distal end 46 in the distal shaft section 43. The distal shaft section 43 has a second lumen 47 for receiving a guide wire 48 over which the delivery catheter is advanced to the desired intracorporeal location for deploying the occluding device. A pusher element 50 having an elongated shaft 51 has an enlarged head 52 on the distal end thereof to engage an occlusion member 10 slidably disposed within the inner lumen 44. The pusher element 50 is long enough so that the proximal end 54 of the shaft 51 extends out of the proximal end 55 of the catheter 40 when the enlarged head 52 thereof has pushed the occlusion member 10 out the discharge port 45 in the distal end 46 of the catheter into a body lumen. The guide wire 47 is slidably disposed within the short guide wire lumen 47 which may be about 0.5 to about 50 cm, preferably about 10 to about 35 cm in length. A distal guide wire port 56 is provided in the distal end 46 of the catheter 40 and a proximal guide wire port 57 is provided a short distance proximal from the distal guide wire port and a substantial distance from the proximal end 55 of the catheter. The guide wire 47 may be of conventional structure with an elongated shaft 58, a tapered distal shaft section 59 and a shapeable spring tip 60 which enables steering the distal end of the guide wire within the patient's body lumen by torquing the proximal end 61 which is configured to extend out of the patient's body.

When delivering the occlusion device 10 by means of a rapid delivery catheter 40, the guide wire 47 is usually advanced through the patient's vaginal canal and uterine cavity and into the patient's fallopian tube with a hysteroscope. The shaped spring tip 60 on the distal end of the guide wire 47 may be used to guide the distal tip into the patient's fallopian tube. The guide wire 47 is advanced until the spring tip 60 is disposed distal to the desired location for the occluding member 10. The rapid exchange delivery catheter 40 may then be advanced over the guide wire until the distal end of the delivery catheter 40 is in an appropriate position for the delivery of the occluding device within the patient's body lumen. The pusher element 50 is then distally advanced until the enlarged head 52 pushes the occluding device 20 out the discharge port 45 in the distal end 46 of the delivery catheter 40. The occlusion device 10 expands upon deployment from the delivery catheter 40 and then the delivery catheter and guide wire 47 may be removed from the patient.

The movement of the pusher rod and occluding device within the catheter, of course is relative. That is, in one application, the enlarged head may be held stationary in the longitudinal direction, and the catheter witht eh occluding device therein may be withdrawn, causing the enlarged head to contact and expel the occluding device from within the catheter. Relative to the body lumen, such as the fallopian tube, however, the occluding device does not move. The catheter that is withdrawn and the occlusive device is laid down in the fallopian tube as the catheter is withdrawn. This has the advantage of allowing the occlusive device to be expelled from the catheter lumen into the fallopian tubes so that the occlusive device does not move in a longitudinal direction within the fallopian tube. Since the occlusive device may consist of several spider segments, and since the first one expelled from within the catheter will often expand and engage the wall of the fallopian tube immediately upon release from the confines of the lumen 44, it may be important not to attempt to push the occlusive device in a longitudinal direction once it has begun to attach to the fallopian tube walls.

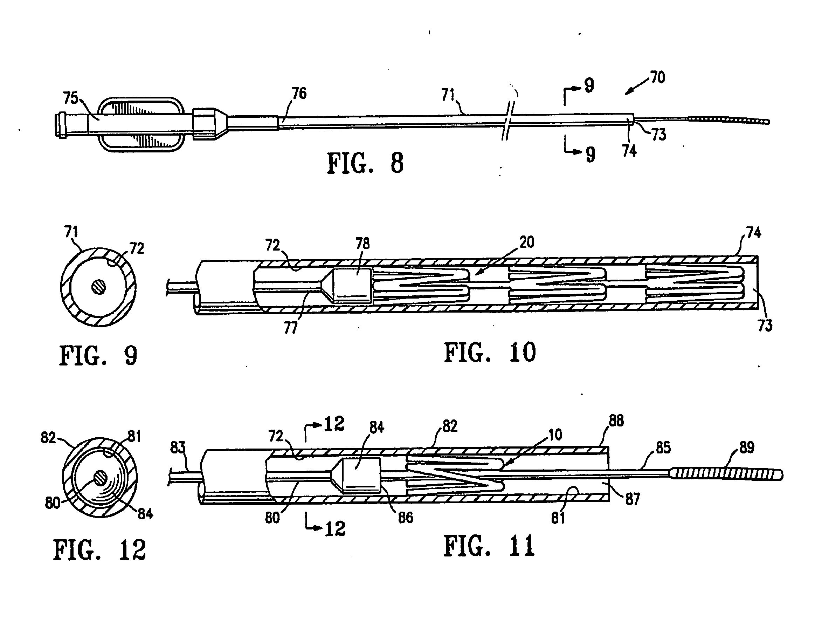

Figure 8-10 depict an over-the-wire type delivery catheter 70 which has an elongated shaft 71, an inner lumen 72, a distal port 73 in the distal end 74 of the shaft and an adapter 75 on the proximal end 76 of the shaft. As shown best in Figure 10 a pusher rod 77 with enlarged head 78 is slidably disposed within the inner lumen 72. The enlarged head 78 is configured to engage the proximal end of occlusion device 20 which is disposed within the inner lumen 72 in a constricted configuration. Distal movement of the pusher rod 77 advances the occlusion device 20 through the inner lumen and out the distal port 73 in the distal end 74.

An alternative delivery system is shown in Figures 11-12 wherein a pusher rod 80 is slidably disposed within an inner lumen 81 of delivery catheter 82. The pusher rod 80 has an elongated shaft 83, an enlarged head 84 and a distal shaft section 85 extending from the front face 86 of the enlarged head 84 is provided with a distal spring tip 86. The pusher rod 80 is in effect a combined pusher rod-guide wire which both guides the delivery system to the desired location and pushes an occlusion device 10 out of the discharge port 87 in the distal end 88 of the delivery catheter 82.

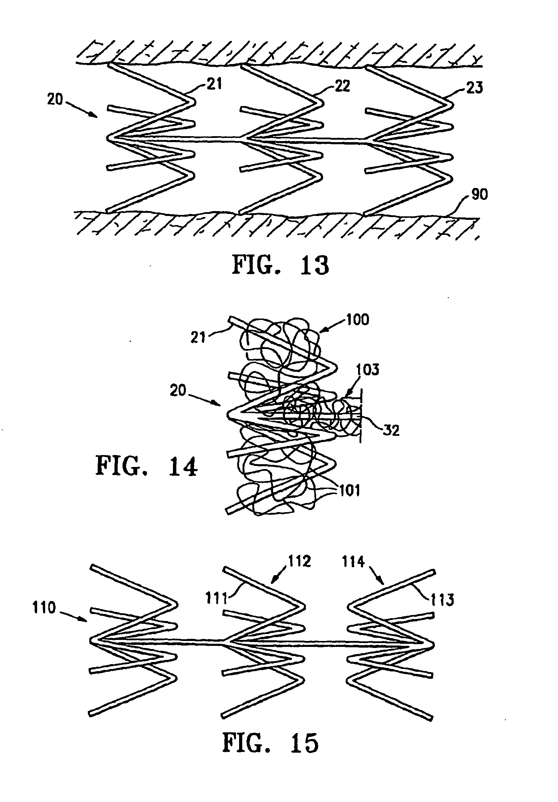

Figure 13 illustrates an occlusion device 20 embodying features of the invention disposed within a patient's body lumen such as a female patient's fallopian tube 90. The spider segments 21, 22 and 23 of the occluding device 20 has expansive elements with free ends which engage the inner lining of the body lumen.

Figure 14 illustrates the proximal portion of occlusion device 20 shown in Figure 3 depicting the expansive elements of spider segment 21 provided with fibrous mass 100 of strands 101 which facilitate tissue ingrowth when the occlusion device is deployed in a female patient's fallopian tube. A similar fibrous mass 103 may be positioned about the connecting beam 32 which extends between the spider segments 21 and the adjacent spider segment 22 (not shown). While fibrous masses of strands are depicted in Figure 14, a variety of materials which facilitate tissue growth within the occluding device to facilitate luminal occlusion may be used to facilitate sufficient tissue ingrowth to effectively occlude the body lumen. The fibrous material is preferably a polyester such as polyethylene terephthalate (PET) Hytrel or a polyamide such as Nylon 6 or ePTFE. Other biocompatible polymeric materials may be employed which facilitate the in-growth of tissue into the device to facilitate effective occlusion of the body lumen. Open cell or closed cell foams or sponges of these or other materials may be used.

Figure 15 illustrates an alternative design for an occlusion device 110 in which the expansive elements 111 of one spider segment 112 are oriented in an opposed orientation to the expansive elements 113 of an adjacent spider segment 114. With the free ends of multiple spider segments in opposing directions, the occluding device 110 is more securely disposed within the patient's body lumen so as to minimize displacement.

Alternatively, drugs and/or hormones may be incorporated within the device in order to accelerate tissue growth into the device, or in or on any of the structural componenets of the occlusive device, or in or on the fibrous masses or strands. Alternatively, if occluding the fallopian tube of a female patient, the device may also elude contraceptive drugs or, if occluding a male patient's reproductive lumen, a spermicide to ensure that the occluding device will be effective immediately upon placement, rather than having to wait for sufficient tissue in-growth into the device for effective occlusion.

The delivery catheter may provide for the delivery of two or more occluding devices. If more than one occluding device is to be delivered within the body (e.g. an occluding device to each fallopian tube), there is no need to remove the initial delivery catheter to deliver additional devices. In such an instance, the physician may deliver one device to the first of two fallopian tubes, and, then access the other fallopian tube with the delivery catheter where the second occluding device is deployed. The use of two occluding devices has the advantage of speeding the overall procedure time and reducing overall costs for the procedure because only one delivery catheter is used.

In another example a length of shaft along the distal end of the delivery catheter is colored a different color than the body of the catheter. As the delivery catheter is advanced through a hysteroscope, the change in color on the distal end of the delivery catheter can be viewed through the hysteroscope as the distal end of the catheter enters the fallopian tube. When the color changed portion disappears from view because it is completely located within the fallopian tube, the enclosed occlusion device is properly located at the specified depth. The occlusion device may then be delivered, ensuring that it is placed at a predetermined depth within the fallopian tube. Depending on the length of the visual marker on the distal end of the delivery catheter, the occlusion device may be located within the isthmic region of the fallopian tube, distal to the isthmic region, or even near the ampulla region of the fallopian tube. An alternative to the variable colored distal region is a visual marker on the delivery catheter. As the visual marker enters the fallopian tube, the occlusion device is at the proper depth for deployment. Alternatively, two markers may be placed to show a prespecified range of depth indication proper placement. Visual markers on the distal end of the delivery catheter may include raised portions or bumps on the exterior of the distal tip of the delivery catheter.

Similarly visual markers such as colored segments, marker lines, or bumps may be located along the length of the guide wire shaft to aid the physician in proper placement of the guide wire. for example, the color bands or other markings may be used to indicate the depth of insertion of the end of the guide wire into the fallopian tubes so that it is properly placed before the Rx catheter is advanced along the guide wire. such markings on the guide wire shaft may also allow the physician to view the guide wire shaft through the hysteroscope and check any movement of the guide wire to prevent inadvertently pushing the guide wire too deep into the fallopian tube when advancing the catheter over the guide wire after the guide wire is initially placed into the fallopian tube.

An alternative to visual means of placement is the use of ultrasound guidance. In this case, a marker that is echogenic is placed on the distal tip of the delivery catheter and a second marker locating the occlusion device within the delivery catheter allows for proper placement of the device under ultrasonic guidance.

Another means of placement for the device is under fluoroscopic guidance. In this case, a radiopaque marker is located at the distal tip of the delivery catheter and a second marker locates the occlusion device within the delivery catheter. When the proper depth of the delivery catheter within the fallopian tube has been seen under fluoroscopy, the occlusion device is ready to be deployed. Additionally, the occlusion device itself may be made radiopaque, either in part or in whole, allowing for direct visualization under fluoroscopy and easier placement.

The device of this invention may be used in the occlusion of various body passageways. For example, the occluding devices of the invention may be used to occlude arteries leading to tumors and other undesirable tissue. Additionally, the devices are particularly well-suited for the steerable delivery of small self expanding intravascular devices, including coronary and neurovascular stents. The devices and methods described herein may be placed using visual means, ultrasonic guidance and/or fluoroscopy.

The occluding members may be preferably formed at least in part of superelastic NiTi alloy with an austenite to martensite transition temperature less than 40°C. preferably less than 25°C. The occlusion device formed at least in part of superelastic NiTi alloy may have the austenite transformed to martensite by reducing the temperature of the device to below the transformation temperature and then constricting the occluding device to facilitate entry into the inner lumen of the delivery catheter in the martensite phase. The mechanical constriction of the occluding device within the delivery catheter maintains the occluding device in the martensite phase. Alternatively, the device may be mechanically compressed to stress-induce the austenite to martensite transformation. When the NiTi devices are released from the delivery catheter, the NiTi alloy transforms from the martensite phase to the more stable, higher strength austenite phase.

Additionally, the occluding devices may be formed at least in part of other high strength biocompatible materials such as MP35N alloy, cobalt-chromium alloys, stainless steel, and high strength biocompatible polymeric materials or combinations thereof may be suitable.

While particular forms of the invention have been illustrated and described herein, it will be apparent to those skilled in the art that various modifications and improvements can be made to the invention. Moreover, individual features of embodiments of the invention may be shown in some drawings and not in others, but those skilled in the art will recognize that individual features of one embodiment of the invention can be combined with any or all the features of another embodiment. Accordingly, it is not intended that the invention be limited to the specific embodiments illustrated. It is therefore intended that this invention to be defined by the scope of the appended claims as broadly as the prior art will permit.

## Claims

1. An occluding device (10) for a patient's reproductive lumen having at least one segment (11, 21, 22, 23) with a first contracted configuration and a second expanded configuration, the at least one segment comprising:
a. a first expansive element (12) having a first end secured to a central location (13) in the occluding device and a second end radially spaced from the first end when in an expanded configuration; and
b. at least one additional expansive element (12) having a first end secured to the central location (13) in the occluding device and a second end radially spaced from the central location in the expanded configuration,
**characterised in that** the occluding device comprises a material (100, 103) to facilitate tissue growth within the occluding member to effect lumen occlusion within the reproductive lumen.

2. The device of claim 1 in which the material to facilitate tissue growth is a fibrous material.

3. The device of claim 1 or 2 in which the material to facilitate tissue growth is a fibrous material deployed about or between the expansive elements of the segment.

4. The device according to claim 1, 2, or 3 in which the material to facilitate tissue growth is a biocompatible polymeric material.

5. The device of any preceding claim wherein at least one of the expansive elements has a first section extending from the first end of the element which is oriented toward a first end of the occluding device and a second section extending to the second end of the expansive element which is oriented toward a second end of the occluding device.

6. The device of claim 5 wherein at least one of the first and second sections of the expansive elements are straight.

7. The device of claim 5 wherein at least one of the first and second sections of the expansive elements are curved.

8. The device of claim 5 wherein a feast the first section of the first expansive element is oriented toward a first end of the occluding device and the first section of the second expansive element is oriented toward a second end of the occluding device.

9. The device of claim 5 wherein the first and additional expansive elements are configured into a spider segment.

10. The device of claim 6 having a plurality of axially aligned spider segments.

11. The device of any preceding claim wherein the expansive elements are self expansive from a constricted configuration to an expanded configuration.

12. The device of claim 11 wherein the expansive elements are formed at least in part of superelastic NiTi alloy which has a stable austenite phase at less than 40°C. and which is in a martensite phase when in a constricted configuration.

13. An occluding device for a patient's reproductive lumen according to any preceding claim, comprising:
a. a first segment (21) having a first expansive element having a first end secured to a central location in the occluding device and a second end radially spaced from the first end when in an expanded configuration and, at least one additional expansive element having a first end secured to the central location in the occluding device and a second end radially spaced from the central location in the expanded configuration; and
b. a second segment (22) axially spaced from the first segment, having a first expansive element having a first end secured to a central location in the occluding device and a second end radially spaced from the first end when in an expanded configuration and at least one additional expansive element having a first end secured to the central location in the occluding device and a second end radially spaced from the central location in the expanded configuration.

14. The occluding device of claim 13 wherein a connecting member (32) interconnects the first segment with an adjacent segment.

15. The occluding device of claim 14 wherein the adjacent segment is the second segment.

16. The occluding device of claim 13, 14 or 15 including at least one intermediate segment which is axially disposed between the first and second segments and which has:
a first expansive element having a first end secured to a central location in the occluding device and a second end radially spaced from the first end when in an expanded configuration and
at least one additional expansive element having a first end secured to the central location in the occluding device and a second end radially spaced from the first end when in the expanded configuration;

17. The occluding device of claim 16 wherein at least one connecting member extends between each segment and an adjacent segment.

18. The occluding device of claim 14 wherein the connecting member is straight.

19. The occluding device of claim 14 wherein the connecting member is curved.

20. An occluding device for a patient's reproductive lumen according to claim 1, comprising:
a. a first segmen (21) which is located at a first end of the occluding device and which has a first expansive element having a first end secured to a central location in the occluding device and a second end radially spaced from the first end when in an expanded configuration and, at least one additional expansive element having a first end secured to the central location in the occluding device and a second end radially spaced from the first end when in an expanded configuration;
b. a second segment (23) which is located at a second end of the occluding device and which has a first expansive element having a first end secured to a central location in the occluding device and a second end radially spaced from the first end when in an expanded configuration and at least one additional expansive element having a first end secured to the central location in the occluding device and a second end radially spaced from the first end when in an expanded configuration;
c. at least one intermediate segment (22) which is axially disposed between the first and second segments and which has: a first expansive element having a first end secured to a central location in the occluding device and a second end radially spaced from the first end when in an expanded configuration and at least one additional expansive element having a first end secured to the central location in the occluding device and a second end radially spaced from the first end when in the expanded configuration;
d. a connecting member (32) extending between and connected to the first segment and an adjacent intermediate segment.

21. The device of claim 20 wherein the first segment and the adjacent intermediate segment are axially aligned.

22. The device of claim 20 wherein the second segment and an adjacent intermediate segment are axially aligned.

23. The device of claim 20 wherein the first, second and intermediate segments are axially aligned.

24. The device of claim 20 wherein the segments are connected to adjacent segments by a connecting member which extends axially along a central location within the occluding member.

25. The device of claim 20 wherein the intermediate segments are interconnected by a connecting member.

26. An occluding device for a patient's reproductive lumen according to claim 1 in which the first and second expansive elements are resilient expansive elements and the occluding member is self-expanding.

## Patentansprüche

1. Okklusionsvorrichtung (10) für ein Reproduktionslumen eines Patienten mit wenigstens einem Segment (11, 21, 22, 23) mit einer ersten zusammengezogenen Konfiguration und einer zweiten gespreizten Konfiguration, wobei das wenigstens eine Segment Folgendes umfasst:
a. ein erstes Spreizelement (12) mit einem ersten, an einer zentralen Position (13) in der Okklusionsvorrichtung befestigten Ende und einem zweiten Ende, das von dem ersten Ende radial beabstandet ist, wenn es in einer gespreizten Konfiguration ist, und
b. wenigstens ein zusätzliches Spreizelement (12) mit einem ersten, an der zentralen Position (13) in der Okklusionsvorrichtung befestigten Ende und einem zweiten Ende hat, das von der zentralen Position radial beabstandet ist, wenn es in der gespreizten Konfiguration ist,
**dadurch gekennzeichnet, dass** die Okklusionsvorrichtung ein Material (100, 103) zur Förderung von Gewebewachstum in dem Okklusionselement aufweist, um die Lumenokklusion in dem Reproduktionslumen zu bewirken.

2. Vorrichtung nach Anspruch 1, bei der das Material zur Förderung von Gewebewachstum ein Fasermaterial ist.

3. Vorrichtung nach Anspruch 1 oder 2, bei dem das Material zur Förderung von Gewebewachstum ein Fasermaterial ist, das um die Spreizelemente des Segments oder zwischen ihnen angeordnet ist.

4. Vorrichtung nach Anspruch 1, 2 oder 3, bei dem das Material zur Förderung von Gewebewachstum ein bioverträgliches polymeres Material ist.

5. Vorrichtung nach einem der vorhergehenden Ansprüche, bei dem wenigstens eines der Spreizelemente einen ersten, sich von dem ersten Ende des Elements erstreckenden Abschnitt, der in Richtung auf ein erstes Ende der Okklusionsvorrichtung ausgerichtet ist, und einen zweiten, sich zu dem zweiten Ende des Spreizelements erstreckenden Abschnitt, der in Richtung auf ein zweites Ende der Okklusionsvorrichtung ausgerichtet ist, hat.

6. Vorrichtung nach Anspruch 5, bei der wenigstens einer der ersten und zweiten Abschnitte der Spreizelemente gerade ist.

7. Vorrichtung nach Anspruch 5, wobei wenigstens einer der ersten und zweiten Abschnitte der Spreizelemente gekrümmt ist.

8. Vorrichtung nach Anspruch 5, bei der wenigstens der erste Abschnitt des ersten Spreizelements in Richtung auf ein erstes Ende der Okklusionsvorrichtung ausgerichtet ist und der erste Abschnitt des zweiten Spreizelements in Richtung auf ein zweites Ende der Okklusionsvorrichtung ausgerichtet ist.

9. Vorrichtung nach Anspruch 5, bei der das erste und zusätzliche Spreizelemente zu einem Spinnenelement konfiguriert sind.

10. Vorrichtung nach Anspruch 6, die eine Vielzahl von axial fluchtenden Spinnensegmenten hat.

11. Vorrichtung nach einem der vorhergehenden Ansprüche, bei der die Spreizelemente selbstspreizend von einer eingeengten Konfiguration auf eine gespreizte Konfiguration sind.

12. Vorrichtung nach Anspruch 11, bei der die Spreizelemente wenigstens teilweise aus hochelastischer NiTi-Legierung ausgebildet sind, die bei weniger als 40°C eine stabile Austenitphase hat und die sich in einer Martensitphase befindet, wenn sie in einer eingeengten Konfiguration sind.

13. Okklusionsvorrichtung für ein Reproduktionslumen eines Patienten nach einem der vorhergehenden Ansprüche, umfassend:
a. ein erstes Segment (21), das ein erstes Spreizelement mit einem ersten, an einer zentralen Position in der Okklusionsvorrichtung befestigten Ende und einem zweiten, von dem ersten Ende radial beabstandeten Ende, wenn es in einer gespreizten Konfiguration ist, und wenigstens ein zusätzliches Spreizelement mit einem ersten, an der zentralen Position in der Okklusionsvorrichtung befestigten Ende und einem zweiten, von der zentralen Position in der gespreizten Konfiguration radial beabstandeten Ende ist, hat und
b. ein von dem ersten Segment axial beabstandetes zweites Segment (22), das ein erstes Spreizelement mit einem ersten, an einer zentralen Position in der Okklusionsvorrichtung befestigten Ende und einem zweiten, von dem ersten Ende radial beabstandeten Ende, wenn es in einer gespreizten Konfiguration ist, und wenigstens ein zusätzliches Spreizelement mit einem ersten, an der zentralen Position in der Okklusionsvorrichtung befestigten Ende und einem zweiten, von der zentralen Position in der gespreizten Konfiguration radial beabstandeten Ende hat.

14. Okklusionsvorrichtung nach Anspruch 13, bei der ein Verbindungselement (32) das erste Segment mit einem benachbarten Segment verbindet.

15. Okklusionsvorrichtung nach Anspruch 14, bei der das benachbarte Segment das zweite Segment ist.

16. Okklusionsvorrichtung nach Anspruch 13, 14 oder 15, das wenigstens ein Zwischensegment aufweist, das axial zwischen dem ersten und dem zweiten Segment angeordnet ist und Folgendes hat:
ein erstes Spreizelement mit einem ersten, an einer zentralen Position in der Okklusionsvorrichtung befestigten Ende und einem zweiten, von dem ersten Ende radial beabstandeten Ende, wenn es in einer gespreizten Konfiguration ist, und
wenigstens ein zusätzliches Spreizelement mit einem ersten, an der zentralen Position in der Okklusionsvorrichtung befestigten Ende und einem zweiten, von dem ersten Ende radial beabstandeten Ende, wenn es in der gespreizten Konfiguration ist.

17. Okklusionsvorrichtung nach Anspruch 16, bei dem sich zwischen jedem Segment und einem benachbarten Segment wenigstens eine Verbindungselement erstreckt.

18. Okklusionsvorrichtung nach Anspruch 14, bei dem das Verbindungselement gerade ist.

19. Okklusionsvorrichtung nach Anspruch 14, bei dem das Verbindungselement gekrümmt ist.

20. Okklusionsvorrichtung für ein Reproduktionslumen eines Patienten nach Anspruch 1, umfassend:
a. ein erstes Segment (21), das sich an einem ersten Ende der Okklusionsvorrichtung befindet und das ein erstes Spreizelement mit einem ersten, an einer zentralen Position in der Okklusionsvorrichtung befestigten Ende und einem zweiten Ende, das von dem ersten Ende radial beabstandet ist, wenn es in einer gespreizten Konfiguration ist, und wenigstens ein zusätzliches Spreizelement mit einem ersten, an der zentralen Position in der Okklusionsvorrichtung befestigten Ende und einem zweiten Ende, das von dem ersten Ende radial beabstandet ist, wenn es in einer gespreizten Konfiguration ist, hat,
b. ein zweites Segment (23), das sich an einem zweiten Ende der Okklusionsvorrichtung befindet und das ein erstes Spreizelement mit einem ersten, an einer zentralen Position in der Okklusionsvorrichtung befestigten Ende und einem zweiten Ende, das von dem ersten Ende radial beabstandet ist, wenn es in einer gespreizten Konfiguration ist, und wenigstens ein zusätzliches Spreizelement mit einem ersten, an der zentralen Position in der Okklusionsvorrichtung befestigten Ende und einem zweiten Ende, das von dem ersten Ende radial beabstandet ist, wenn es in der gespreizten Konfiguration ist, hat,
c. wenigstens ein Zwischenelement (22), das axial zwischen dem ersten und dem zweiten Segment angeordnet ist und das Folgendes hat: ein erstes Spreizelement mit einem ersten, an einer zentralen Position in der Okklusionsvorrichtung befestigten Ende und einem zweiten, von dem ersten Ende radial beabstandeten Ende, wenn es in einer gespreizten Konfiguration ist, und wenigstens ein zusätzliches Spreizelement mit einem ersten, an der zentralen Position in der Okklusionsvorrichtung befestigten Ende und einem zweiten, von dem ersten Ende radial beabstandeten Ende, wenn es in der gespreizten Konfiguration ist,
d. ein Verbindungselement (32), das sich zwischen dem ersten Segment und einem benachbarten Zwischensegment befindet und mit diesen verbunden ist.

21. Vorrichtung nach Anspruch 20, bei der das erste Segment und das benachbarte Zwischensegment axial fluchten.

22. Vorrichtung nach Anspruch 20, bei der das zweite Segment und ein benachbartes Zwischensegment axial fluchten.

23. Vorrichtung nach Anspruch 20, bei der das erste, das zweite und das Zwischensegment axial fluchten.

24. Vorrichtung nach Anspruch 20, bei der die Segmente durch ein Verbindungselement, das entlang einer zentralen Position in dem Okklusionselement verläuft, mit benachbarten Segmenten verbunden sind.

25. Vorrichtung nach Anspruch 20, bei der die Zwischensegmente von einem Verbindungselement miteinander verbunden sind.

26. Okklusionsvorrichtung für ein Reproduktionslumen eines Patienten nach Anspruch 1, bei dem die ersten und zweiten Spreizelemente elastische Spreizelemente sind und das Okklusionselement selbstspreizend ist.

## Revendications

1. Dispositif d'occlusion (10) d'une lumière de reproduction d'un patient ayant au moins un segment (11, 21, 22, 23) avec une première configuration contractée et une seconde configuration expansée, l'au moins un segment comprenant :
a. un premier élément expansif (12) ayant une première extrémité fixée à un emplacement central (13) dans le dispositif d'occlusion et une seconde extrémité espacée radialement de la première extrémité lorsqu'il est en configuration expansée ; et
b. au moins un élément expansif supplémentaire (12) ayant une première extrémité fixée à l'emplacement central (13) dans le dispositif d'occlusion et une seconde extrémité espacée radialement de l'emplacement central dans la configuration expansée,
**caractérisé en ce que** le dispositif d'occlusion comprend un matériau (100, 103) pour faciliter la croissance de tissu dans l'élément occlusif et effectuer une occlusion de lumière dans la lumière de reproduction.

2. Dispositif selon la revendication 1, dans lequel le matériau pour faciliter la croissance de tissu est un matériau fibreux.

3. Dispositif selon la revendication 1 ou 2, dans lequel la matériau pour faciliter la croissance de tissu est un matériau fibreux déployé autour des éléments expansifs du segment ou entre ceux-ci.

4. Dispositif selon la revendication 1, 2 ou 3, dans lequel le matériau pour faciliter la croissance de tissu est un matériau polymérique biocompatible.

5. Dispositif selon l'une quelconque des revendications précédentes, dans lequel au moins l'un des éléments expansifs a une première section qui s'étend depuis la première extrémité de l'élément qui est orientée vers une première extrémité du dispositif d'occlusion et une seconde section qui s'étend jusqu'à la seconde extrémité de l'élément expansif qui est orientée vers une seconde extrémité du dispositif d'occlusion.

6. Dispositif selon la revendication 5, dans lequel au moins l'une des première et seconde sections des éléments expansifs est droite.

7. Dispositif selon la revendication 5, dans lequel au moins l'une des première et seconde sections des éléments expansifs est courbe.

8. Dispositif selon la revendication 5, dans lequel au moins la première section du premier élément expansif est orientée vers une première extrémité du dispositif d'occlusion et la première section du second élément expansif est orientée vers une seconde extrémité du dispositif d'occlusion.

9. Dispositif selon la revendication 5, dans lequel les premier et second éléments expansifs sont configurés en un segment en forme d'araignée.

10. Dispositif selon la revendication 6, ayant une pluralité de segments en forme d'araignée alignés axialement.

11. Dispositif selon l'une quelconque des revendications précédentes, dans lequel les éléments expansifs sont auto-expansifs d'une configuration contrainte à une configuration expansée.

12. Dispositif selon la revendication 11, dans lequel les éléments expansifs sont formés au moins en partie en un alliage NiTi superélastique qui a une phase austénique stable à moins de 40°C et qui est en phase martensitique dans une configuration contrainte.

13. Dispositif d'occlusion d'une lumière de reproduction d'un patient selon l'une quelconque des revendications précédentes, comprenant :
a. un premier segment (21) ayant un premier élément expansif ayant une première extrémité fixée à un emplacement central dans le dispositif d'occlusion et une seconde extrémité espacée radialement de la première extrémité lorsqu'il est en configuration expansée, et au moins un élément expansif supplémentaire ayant une première extrémité fixée à un emplacement central dans le dispositif d'occlusion et une seconde extrémité espacée radialement de l'emplacement central dans la configuration expansée ; et
b. un second segment (22) espacé axialement du premier segment, ayant un premier élément expansif ayant une première extrémité fixée à un emplacement central dans le dispositif d'occlusion et une seconde extrémité espacée radialement de la première extrémité lorsqu'il est en configuration expansée, et au moins un élément expansif supplémentaire ayant une première extrémité fixée à un emplacement central dans le dispositif d'occlusion et une seconde extrémité espacée radialement de l'emplacement central dans la configuration expansée.

14. Dispositif d'occlusion selon la revendication 13, dans lequel un élément de connexion (32) interconnecte le premier segment à un segment adjacent.

15. Dispositif d'occlusion selon la revendication 14, dans lequel le segment adjacent est le second segment.

16. Dispositif d'occlusion selon la revendication 13, 14 ou 15, comportant au moins un segment intermédiaire qui est disposé axialement entre les premier et second segments et qui a :
un premier élément expansif ayant une première extrémité fixée à un emplacement central dans le dispositif d'occlusion et une seconde extrémité espacée radialement de la première extrémité lorsqu'il est en configuration expansée et
au moins un élément expansif supplémentaire ayant une première extrémité fixée à un emplacement central dans le dispositif d'occlusion et une seconde extrémité espacée radialement de la première extrémité lorsqu'il est en configuration expansée.

17. Dispositif d'occlusion selon la revendication 16, dans lequel au moins un élément de connexion s'étend entre chaque segment et un segment adjacent.

18. Dispositif d'occlusion selon la revendication 14, dans lequel l'élément de connexion est droit.

19. Dispositif d'occlusion selon la revendication 14, dans lequel l'élément de connexion est courbe.

20. Dispositif d'occlusion d'une lumière de reproduction d'un patient selon la revendication 1, comprenant :
a. un premier segment (21) situé à une première extrémité du dispositif d'occlusion et ayant un premier élément expansif ayant une première extrémité fixée à un emplacement central dans le dispositif d'occlusion et une seconde extrémité espacée radialement de la première extrémité lorsqu'il est en configuration expansée, et au moins un élément expansif supplémentaire ayant une première extrémité fixée à un emplacement central dans le dispositif d'occlusion et une seconde extrémité espacée radialement de l'emplacement central lorsqu'il est en configuration expansée ; et
b. un second segment (22) situé à une seconde extrémité du dispositif d'occlusion et ayant un premier élément expansif ayant une première extrémité fixée à un emplacement central dans le dispositif d'occlusion et une seconde extrémité espacée radialement de la première extrémité lorsqu'il est en configuration expansée, et au moins un élément expansif supplémentaire ayant une première extrémité fixée à un emplacement central dans le dispositif d'occlusion et une seconde extrémité espacée radialement de la première extrémité lorsqu'il est en configuration expansée ;
c. au moins un segment intermédiaire (22) disposé axialement entre les premier et second segments et ayant : un premier élément expansif ayant une première extrémité fixée à un emplacement central dans le dispositif d'occlusion et une seconde extrémité espacée radialement de la première extrémité lorsqu'il est en configuration expansée et au moins un élément expansif supplémentaire ayant une première extrémité fixée à l'emplacement central dans le dispositif d'occlusion et une seconde extrémité espacée radialement de la première extrémité lorsqu'il est en configuration expansée ;
d. un élément de connexion (32) s'étendant entre le premier segment et un segment intermédiaire adjacent et connecté à ceux-ci.

21. Dispositif selon la revendication 20, dans lequel le premier segment et le segment intermédiaire adjacent sont alignés axialement.

22. Dispositif selon la revendication 20, dans lequel le second segment et un segment intermédiaire adjacent sont alignés axialement.

23. Dispositif selon la revendication 20, dans lequel le premier segment, le second segment et le segment intermédiaire sont alignés axialement.

24. Dispositif selon la revendication 20, dans lequel les segments sont connectés à des segments adjacents par un élément de connexion qui s'étend axialement le long d'un emplacement central dans l'élément occlusif.

25. Dispositif selon la revendication 20, dans lequel les segments intermédiaires sont interconnectés par un élément de connexion.

26. Dispositif d'occlusion d'une lumière de reproduction d'un patient selon la revendication 1, dans lequel les premier et second éléments expansifs sont des éléments expansifs résilients et l'élément occlusif est auto-expansif.
